# EUROPÄISCHE PATENTSCHRIFT

(11) **EP 3 450 592 B1**
(45) Veröffentlichungstag und Bekanntmachung des Hinweises auf die Patenterteilung: **25.03.2020**
(21) Anmeldenummer: 17188057.8
(22) Anmeldetag: 28.08.2017
(51) Int. Cl.: C25B 3/10, C07D 333/16

(54) **ELEKTROCHEMISCHE KUPPLUNG VON PHENOLEN ÜBER THIOPHEN**
ELECTROCHEMICAL COUPLING OF PHENOLS VIA THIOPHENE
COUPLAGE ÉLECTROCHIMIQUE DES PHÉNOLS AU THIOPHÈNE

(43) Veröffentlichungstag der Anmeldung: 06.03.2019
(73) Patentinhaber: Evonik Operations GmbH, 45128 Essen (DE)
(72) Erfinder: WALDVOGEL, Siegfried R., 55435 Gau-Algesheim (DE); WIEBE, Anton, 56567 Neuwied (US); LIPS, Sebastian, 55595 Hüffelsheim (DE); FRANKE, Robert, 45772 Marl (DE)
(74) Vertreter: Evonik Patent Association

(56) Entgegenhaltungen:
- WO-A1-2014/135371
- TATSUYA MOROFUJI ET AL: "Metal- and Chemical-Oxidant-Free C-H/C-H Cross-Coupling of Aromatic Compounds: The Use of Radical-Cation Pools", ANGEWANDTE CHEMIE INTERNATIONAL EDITION, Bd. 51, Nr. 29, 16. Juli 2012 (2012-07-16) , Seiten 7259-7262, XP055426856, ISSN: 1433-7851, DOI: 10.1002/anie.201202788
- BERND ELSLER ET AL: "Source of Selectivity in Oxidative Cross-Coupling of Aryls by Solvent Effect of 1,1,1,3,3,3-Hexafluoropropan-2-ol", CHEMISTRY - A EUROPEAN JOURNAL, Bd. 21, Nr. 35, 16. Juli 2015 (2015-07-16) , Seiten 12321-12325, XP055426869, ISSN: 0947-6539, DOI: 10.1002/chem.201501604

## Beschreibung

Die Erfindung betrifft ein Verfahren zur elektrochemischen Kupplung von Phenolen über Thiophen.

Elektrochemische Verfahren zur Kreuzkupplung von Phenolen über Thiophen sind bisher nicht bekannt.

Klassische Verfahren der Kreuzkupplung mit Thiophenen sind vor allem auf übergangsmetallkatalysierte Reaktionen, wie Beispielsweise Suzuki-, Stille- und Negishi-Kupplungen konzentriert. Bei diesen Verfahren müssen die Hydroxygruppen der Phenole häufig geschützt werden (meist durch O-Methylierung). Genannte Reaktionen erfordern Abgangsfunktionalitäten in beiden Substraten, sowie eine anaerobe Reaktionsführung. Klassische Kreuzkupplungsreaktionen werden unter anaeroben Bedingungen und teilweise unter Feuchtigkeitsausschluss durchgeführt. Des Weiteren machen knapper werdende Rohstoffe den Einsatz von Palladium-basierten Katalysatoren sehr kostenintensiv.

In WO 2014/135371 A1 wird ein elektrochemisches Verfahren zur Kupplung von Phenol und Anilin beschreiben.

In Tatsuya Morofuji et al. "Metal- and chemical-oxidant-free C-H/C-H cross-coupling of aromatic compounds: the use of radical-cation pools.", Angew Chem Int Ed Engl., Bd. 51, Nr. 29, 16. Juli 2012, Seiten 7259-7262, wird eine C-H/C-H Kreuzkupplung von Aromaten beschreiben. Diese erfolgt mit der "radical-cation pool" Methode.

In Bernd Elsler et al. "Source of Selectivity in Oxidative Cross-Coupling of Aryls by Solvent Effect of 1,1,1,3,3,3-Hexafluoropropan-2-ol", Chemistry - a European Journal, Bd. 21, Nr. 35, 16. Juli 2015, Seiten 12321 - 12325, wird der Einsatz von 1,1,1,3,3,3-Hexafluoropropan-2-ol als Lösungsmittel bei Kreuzkupplungsreaktionen beschreiben.

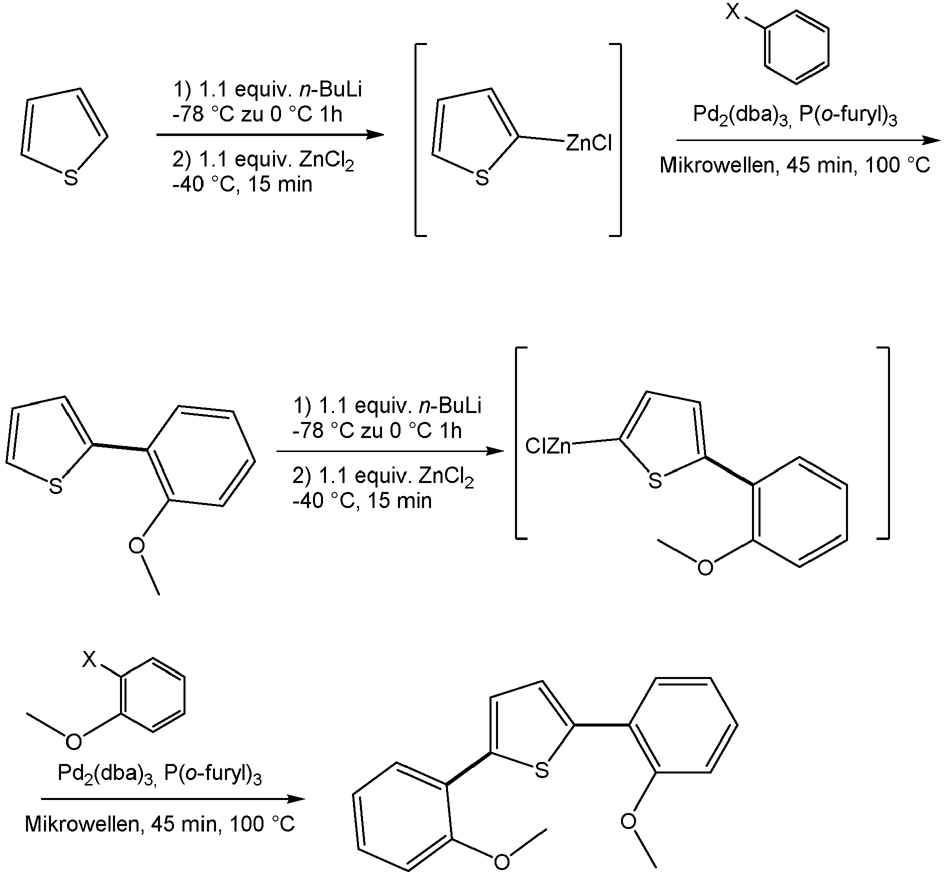

Der Erfindung lag die Aufgabe zugrunde, ein Verfahren bereitzustellen, welches die in Zusammenhang mit dem Stand der Technik diskutierten Nachteile nicht aufweist.

Gelöst wird die Aufgabe durch das erfindungsgemäße Verfahren.

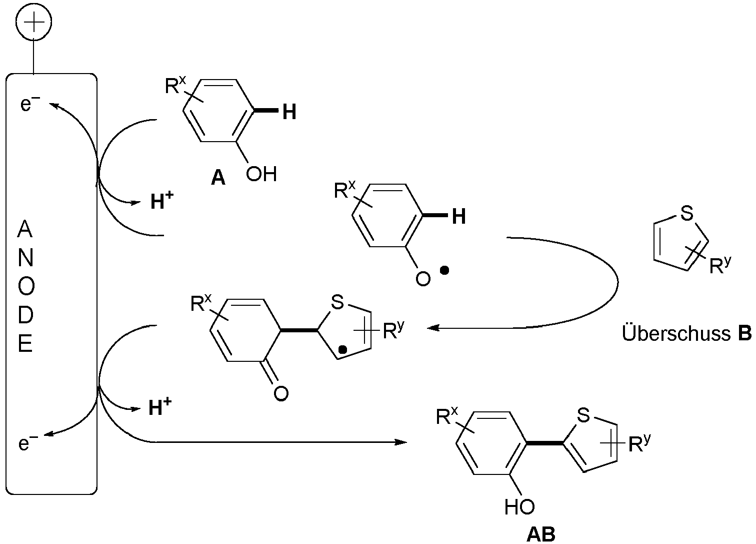

Im verwendeten Lösungsmittel (1,1,1,3,3,3-Hexafluorisopropanol) wird die Komponente A selektiv oxidiert. Die gebildete Radikalspezies wird vom Lösungsmittel stabilisiert und kann auf diese Weise selektiv nukleophil von Komponente B (Thiophen) angegriffen werden.

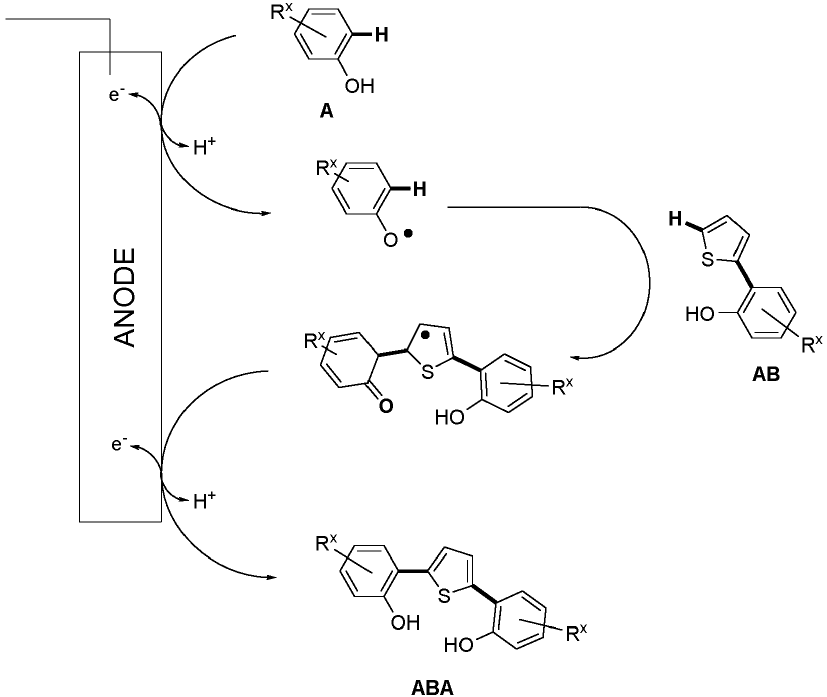

Nach demselben Prinzip verläuft die Bildung der entsprechenden Verbindungen **ABA.** Nach Entstehung der Phenol-Thiophen-Verbindungen **AB** fungiert diese hier als Nukleophil angreifende Spezies am Phenoxylradikal.

Das erfindungsgemäße Verfahren ermöglicht erstmals die Verwendung von Heterozyklen unter den Bedingungen der anodischen C-C-Kreuzkupplung.

Verfahren umfassend die Verfahrensschritte:
a) Zugabe einer Verbindung der allgemeinen Formel (**I**): 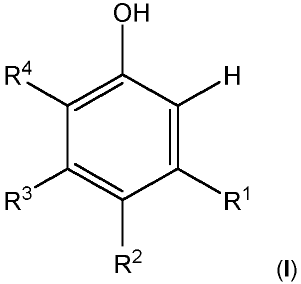 wobei R¹, R², R³, R⁴ jeweils unabhängig voneinander ausgewählt sind aus: -H, -(C₁-C₁₂)-Alkyl,-O-(C₁-C₁₂)-Alkyl, -(C₄-C₁₄)-Aryl, -O-(C₄-C₁₄)-Aryl, Cycloalkyl,
   wobei die genannten Alkylgruppen, Arylgruppen, Cycloalkyl, wie folgt substituiert sein können: -(C₁-C₁₂)-Alkyl, -O-(C₁-C₁₂)-Alkyl;
b) Zugabe einer Verbindung der allgemeinen Formel (**II**): 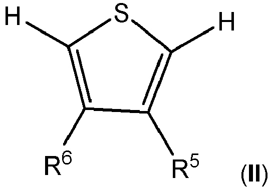 wobei R⁵, R⁶ jeweils unabhängig voneinander ausgewählt sind aus: -H, -(C₁-C₁₂)-Alkyl, -O-(C₁-C₁₂)-Alkyl, -(C₄-C₁₄)-Aryl, -O-(C₄-C₁₄)-Aryl, Cycloalkyl,
   wobei die genannten Alkylgruppen, Arylgruppen, Cycloalkyl, wie folgt substituiert sein können: -(C₁-C₁₂)-Alkyl, -O-(C₁-C₁₂)-Alkyl;
c) Zugabe von 1,1,1,3,3,3-Hexafluorisopropanol;
d) Einbringen zweier Elektroden in die Reaktionslösung;
e) Anlegen von Spannung an die Elektroden, so dass eine Kupplungsreaktion zwischen der Verbindung (**I**) und der Verbindung (**II**) erfolgt.

In einer Variante des Verfahrens ist das Verhältnis von der Verbindung (**I**) zur Verbindung (**II**) so gewählt, dass die Verbindung (**I**) zweimal mit der Verbindung (**II**) reagiert.

In einer Variante des Verfahrens wird das Produkt aus der ersten Kupplungsreaktion (**III**): 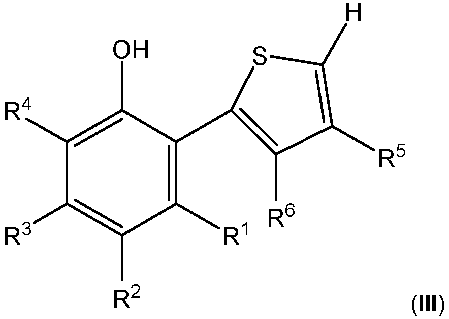 erneut mit der Verbindung (**I**) zur Verbindung (**IV**) umgesetzt:

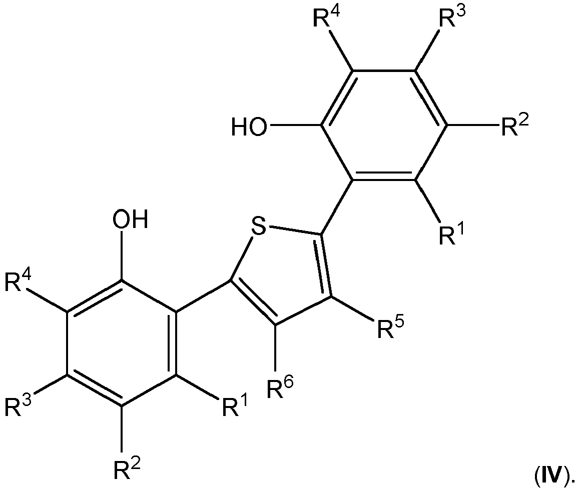

In einer Variante des Verfahrens wird das Produkt aus der ersten Kupplungsreaktion (**III**): 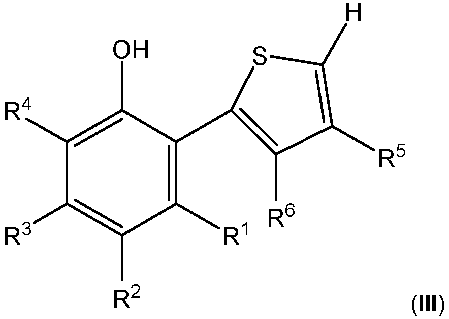 mit der Verbindung (**V**) 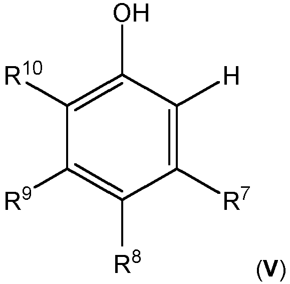 wobei R⁷, R⁸, R⁹, R¹⁰ jeweils unabhängig voneinander ausgewählt sind aus: -H, -(C₁-C₁₂)-Alkyl,-O-(C₁-C₁₂)-Alkyl, -(C₄-C₁₄)-Aryl, -O-(C₄-C₁₄)-Aryl, Cycloalkyl, wobei die genannten Alkylgruppen, Arylgruppen, Cycloalkyl, wie folgt substituiert sein können: -(C₁-C₁₂)-Alkyl, -O-(C₁-C₁₂)-Alkyl;
wobei sich die Verbindung (**V**) in mindestens einem Rest von der Verbindung (**I**) unterscheidet, zur Verbindung (**VI**) umgesetzt:

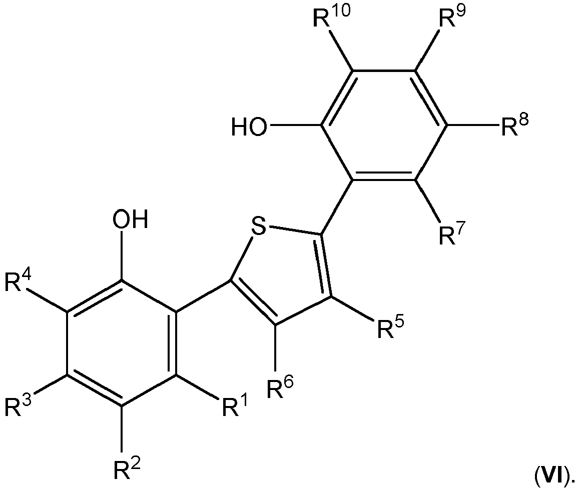

In einer Variante des Verfahrens umfasst das Verfahren den zusätzlichen Verfahrensschritt f): f) Zugabe von Methanol.
In einer Variante des Verfahrens handelt es sich der Anode um eine BDD-Anode. (BDD = Bor-dotierter Diamant)
In einer Variante des Verfahrens handelt es sich der Kathode um eine BDD-Kathode. (BDD = Bor-dotierter Diamant)
In einer Variante des Verfahrens umfasst das Verfahren den zusätzlichen Verfahrensschritt g): g) Zugabe von MTBS.
   (MTBS = [Bu₃NMe]O₃SOMe)
Im Folgenden wird die Erfindung anhand von Ausführungsbeispielen näher erläutert. (HFIP = 1,1,1,3,3,3-Hexafluorisopropanol)

### Einfache Kupplung

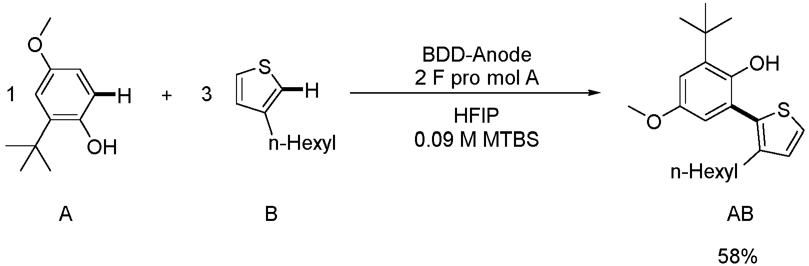

### Doppelte Kupplung (symmetrisch)

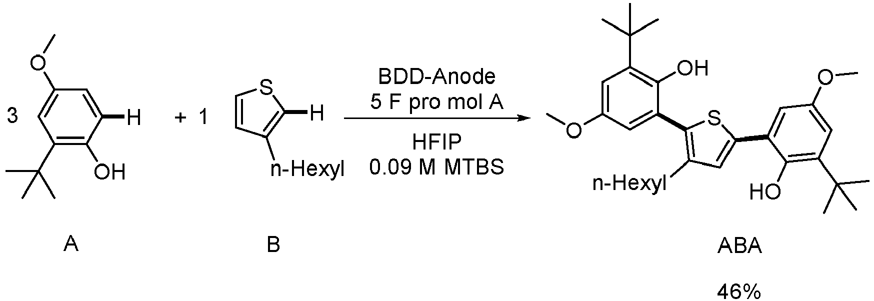

### Doppelte Kupplung (unsymmetrisch)

Die Darstellung unsymmetrischer Pincerliganden durch eine zweistufige Reaktionsführung ist ebenfalls möglich. Hierbei kann zusätzlich die Verwendung von Schutzgruppen (OPG) zu teilgeschützten m-Terarylen führen.

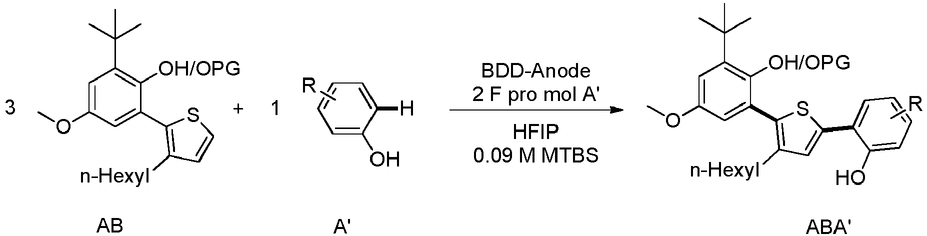

Wie die oben beschriebenen Versuche zeigen, wird die Aufgabe durch ein erfindungsgemäßes Verfahren gelöst.

## Patentansprüche

1. Verfahren umfassend die Verfahrensschritte:
a) Zugabe einer Verbindung der allgemeinen Formel (**I**): 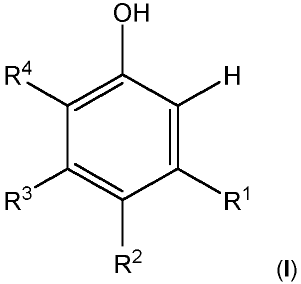 wobei R¹, R², R³, R⁴ jeweils unabhängig voneinander ausgewählt sind aus: -H, -(C₁-C₁₂)-Alkyl,-O-(C₁-C₁₂)-Alkyl, -(C₄-C₁₄)-Aryl, -O-(C₄-C₁₄)-Aryl, Cycloalkyl,
wobei die genannten Alkylgruppen, Arylgruppen, Cycloalkyl, wie folgt substituiert sein können: -(C₁-C₁₂)-Alkyl, -O-(C₁-C₁₂)-Alkyl;
b) Zugabe einer Verbindung der allgemeinen Formel (**II**): 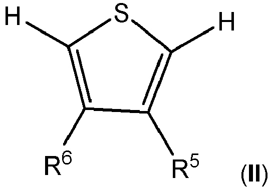 wobei R⁵, R⁶ jeweils unabhängig voneinander ausgewählt sind aus: -H, -(C₁-C₁₂)-Alkyl, -O-(C₁-C₁₂)-Alkyl, -(C₄-C₁₄)-Aryl, -O-(C₄-C₁₄)-Aryl, Cycloalkyl,
wobei die genannten Alkylgruppen, Arylgruppen, Cycloalkyl, wie folgt substituiert sein können: -(C₁-C₁₂)-Alkyl, -O-(C₁-C₁₂)-Alkyl;
c) Zugabe von 1,1,1,3,3,3-Hexafluorisopropanol;
d) Einbringen zweier Elektroden in die Reaktionslösung;
e) Anlegen von Spannung an die Elektroden, so dass eine Kupplungsreaktion zwischen der Verbindung (**I**) und der Verbindung (**II**) erfolgt.

2. Verfahren nach Anspruch 1,
wobei das Verhältnis von der Verbindung (**I**) zur Verbindung (**II**) so gewählt ist, dass die Verbindung (**I**) zweimal mit der Verbindung (**II**) reagiert.

3. Verfahren nach Anspruch 1,
wobei das Produkt aus der ersten Kupplungsreaktion (**III**): 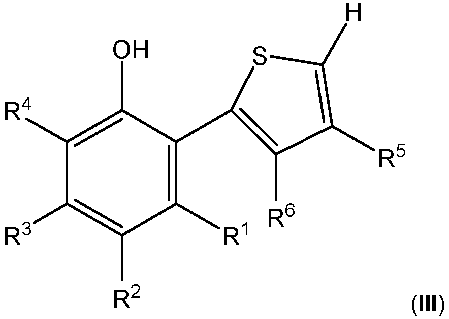 erneut mit der Verbindung (**I**) zur Verbindung (**IV**) umgesetzt wird:

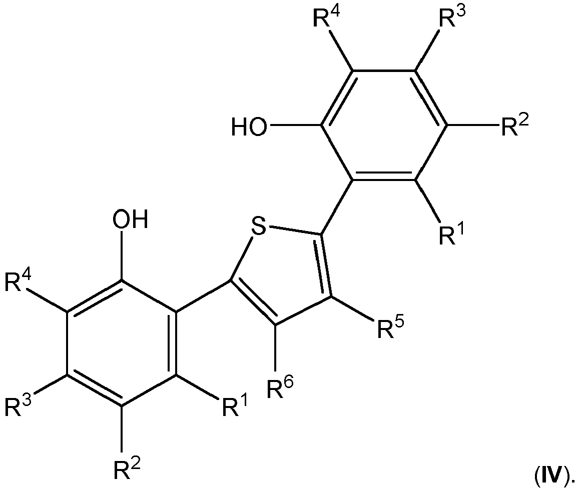

4. Verfahren nach Anspruch 1,
wobei das Produkt aus der ersten Kupplungsreaktion (**III**): 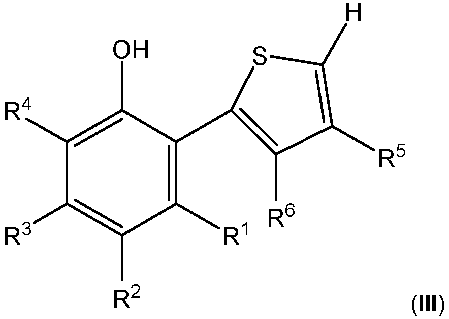 mit der Verbindung (V) 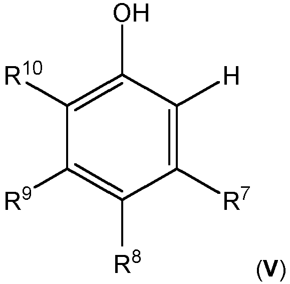 wobei R⁷, R⁸, R⁹, R¹⁰ jeweils unabhängig voneinander ausgewählt sind aus: -H, -(C₁-C₁₂)-Alkyl,-O-(C₁-C₁₂)-Alkyl, -(C₄-C₁₄)-Aryl, -O-(C₄-C₁₄)-Aryl, Cycloalkyl,
wobei die genannten Alkylgruppen, Arylgruppen, Cycloalkyl, wie folgt substituiert sein können: -(C₁-C₁₂)-Alkyl, -O-(C₁-C₁₂)-Alkyl;
wobei sich die Verbindung (**V**) in mindestens einem Rest von der Verbindung (**I**) unterscheidet, zur Verbindung (**VI**) umgesetzt wird:

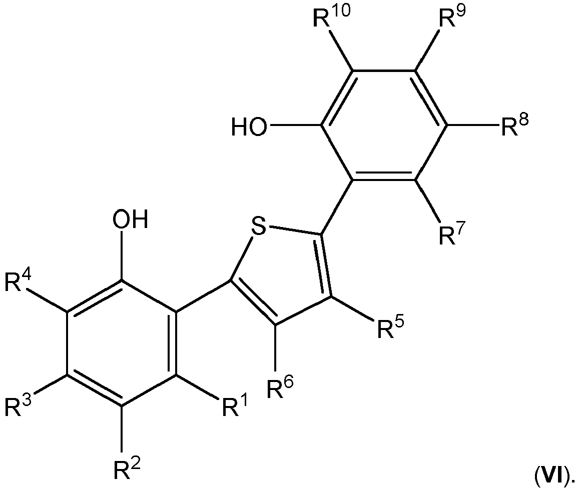

5. Verfahren nach einem der Ansprüche 1 bis 4,
wobei das Verfahren den zusätzlichen Verfahrensschritt f) umfasst: f) Zugabe von Methanol.

6. Verfahren nach einem der Ansprüche 1 bis 5,
wobei es sich der Anode um eine BDD-Anode handelt.

7. Verfahren nach einem der Ansprüche 1 bis 6,
wobei es sich der Kathode um eine BDD-Kathode handelt.

8. Verfahren nach einem der Ansprüche 1 bis 7,
wobei das Verfahren den zusätzlichen Verfahrensschritt g) umfasst: g) Zugabe von MTBS.

## Claims

1. Process comprising the following process steps:
a) adding a compound of the general formula (I): 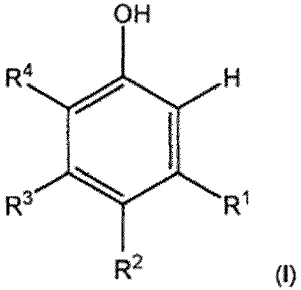 wherein R¹, R², R³, R⁴ are each independently selected from: -H, -(C₁-C₁₂) alkyl, -O-(C₁-C₁₂) alkyl, -(C₄-C₁₄) aryl, -O-(C₄-C₁₄) aryl, cycloalkyl, mentioned wherein the alkyl, aryl and cycloalkyl groups may be substituted as follows:
-(C₁-C₁₂) alkyl, -O-(C₁-C₁₂) alkyl;
b) adding a compound of the general formula (**II**): 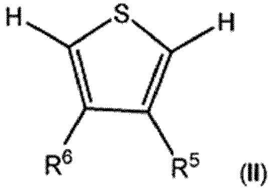 wherein R⁵, R⁶ are each independently selected from: -H, -(C₁-C₁₂) alkyl, -O-(C₁-C₁₂) alkyl, -(C₄-C₁₄) aryl, -O-(C₄-C₁₄) aryl, cycloalkyl,
wherein the alkyl, aryl and cycloalkyl groups mentioned may be substituted as follows:
-(C₁-C₁₂) alkyl, -O-(C₁-C₁₂) alkyl;
c) adding 1,1,1,3,3,3-hexafluoroisopropanol;
d) introducing two electrodes into the reaction solution;
e) applying a voltage at the electrodes so that a coupling reaction takes place between compound (**I**) and compound (**II**).

2. Process according to Claim 1,
wherein the ratio of compound (**I**) to compound (**II**) is chosen so that compound (**I**) reacts twice with compound (**II**).

3. Process according to Claim 1,
wherein the product of the first coupling reaction (**III**): 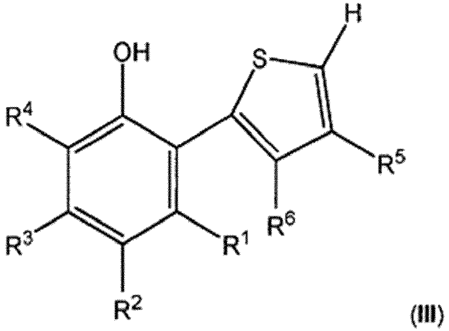 is reacted again with compound (**I**) to form compound (**IV**):

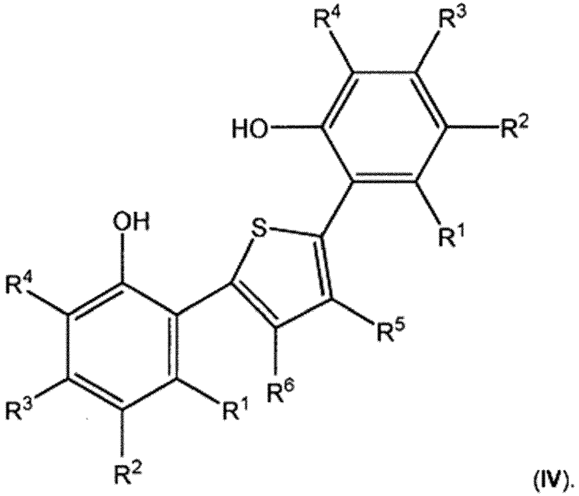

4. Process according to Claim 1,
wherein the product of the first coupling reaction (**III**): 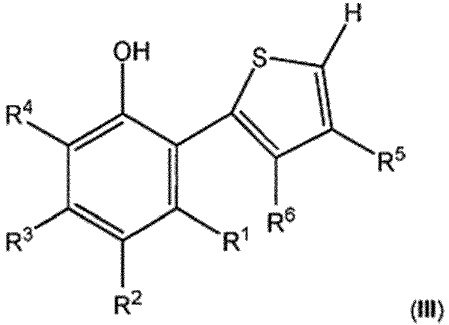 is reacted with compound (**V**) 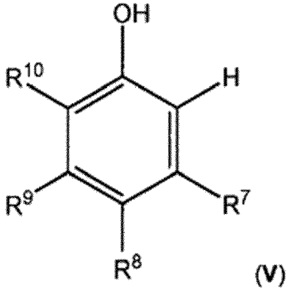 wherein R⁷, R⁸, R⁹, R¹⁰ are each independently selected from: -H, -(C₁-C₁₂) alkyl, -O-(C₁-C₁₂) alkyl, -(C₄-C₁₄) aryl, -O-(C₄-C₁₄) aryl, cycloalkyl,
wherein the alkyl, aryl and cycloalkyl groups mentioned may be substituted as follows:
-(C₁-C₁₂) alkyl, -O-(C₁-C₁₂) alkyl;
wherein compound (**V**) differs in at least one radical from compound (**I**), to form compound (**VI**):

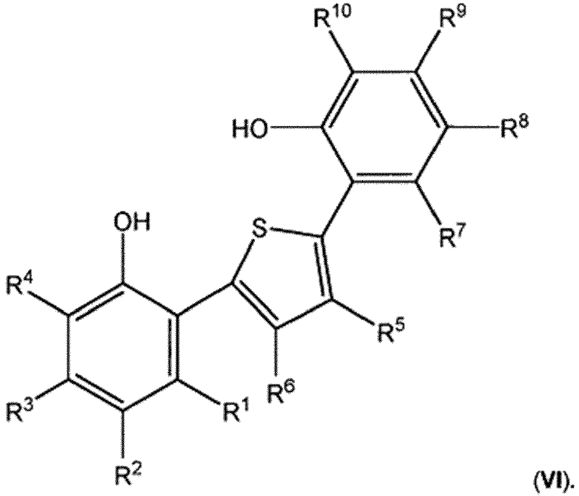

5. Process according to any of Claims 1 to 4,
wherein the process includes the additional process step f):
f) adding methanol.

6. Process according to any of Claims 1 to 5,
wherein the anode is a BDD anode.

7. Process according to any of Claims 1 to 6,
wherein the anode is a BDD cathode.

8. Process according to any of Claims 1 to 7,
wherein the process includes the additional process step g):
g) adding MTBS.

## Revendications

1. Procédé comprenant les étapes de procédé :
a) ajout d'un composé de formule générale (I) : 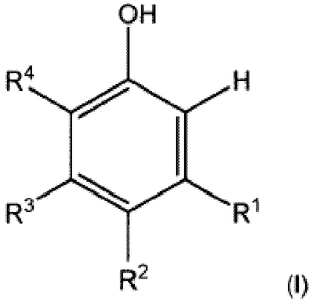 R¹, R², R³, R⁴ étant à chaque fois choisis indépendamment les uns des autres parmi : -H,-C₁₋₁₂-alkyle, -O-C₁₋₁₂-alkyle, -C₄₋₁₄-aryle, -O-C₄₋₁₄-aryle, cycloalkyle,
les groupes alkyle, les groupes aryle, cycloalkyle mentionnés pouvant être substitués comme suit :
-C₁₋₁₂-alkyle, -O-C₁₋₁₂-alkyle ;
b) ajout d'un composé de formule générale (II) : 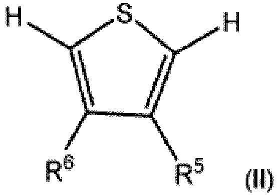 R⁵, R⁶ étant à chaque fois choisis indépendamment l'un de l'autre parmi : -H, -C₁₋₁₂-alkyle, -O-C₁₋₁₂-alkyle, -C₄₋₁₄-aryle, -O-C₄₋₁₄-aryle, cycloalkyle,
les groupes alkyle, les groupes aryle, cycloalkyle mentionnés pouvant être substitués comme suit :
-C₁₋₁₂-alkyle, -O-C₁₋₁₂-alkyle ;
c) ajout de 1,1,1,3,3,3-hexafluoroisopropanol;
d) introduction de deux électrodes dans la solution de réaction ;
e) application de tension au niveau des électrodes, afin de réaliser une réaction de couplage entre le composé (I) et le composé (II) .

2. Procédé selon la revendication 1,
le rapport du composé (I) au composé (II) étant choisi de sorte que le composé (I) réagisse deux fois avec le composé (II).

3. Procédé selon la revendication 1,
le produit de la première réaction de couplage (III) : 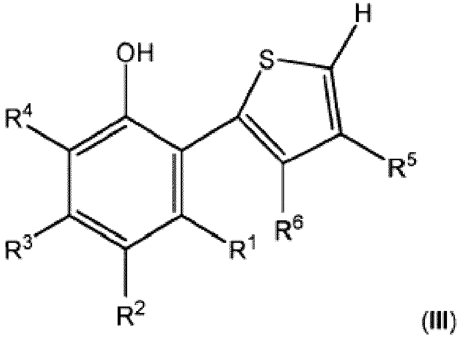 étant transformé à nouveau avec le composé (I) en le composé (IV) :

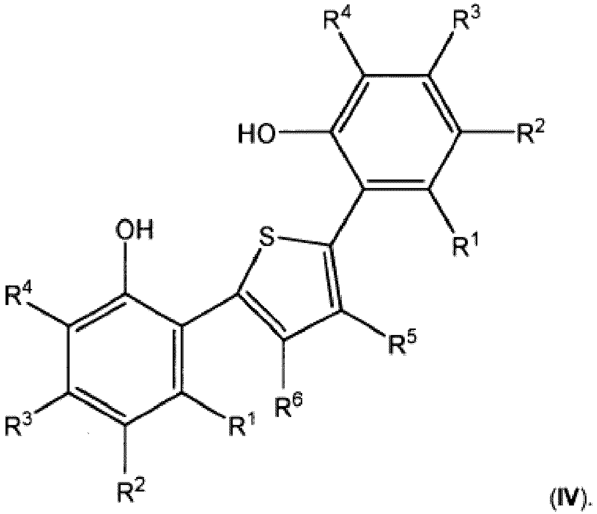

4. Procédé selon la revendication 1,
le produit de la première réaction de couplage (III) : 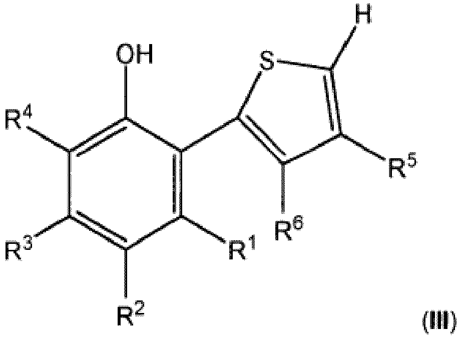 étant transformé avec le composé (V) 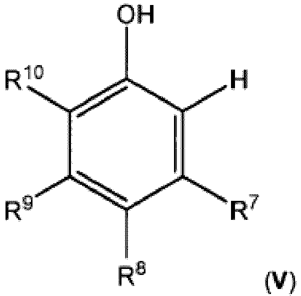 R⁷, R⁸, R⁹, R¹⁰ étant à chaque fois choisis indépendamment les uns des autres parmi : -H, -C₁₋₁₂-alkyle, -O-C₁₋₁₂-alkyle, -C₄₋₁₄-aryle, -O-C₄₋₁₄-aryle, cycloalkyle,
les groupes alkyle, les groupes aryle, cycloalkyle mentionnés pouvant être substitués comme suit :
-C₁₋₁₂-alkyle, -O-C₁₋₁₂-alkyle ;
le composé (V) se différenciant du composé (I) au niveau d'au moins un radical,
en le composé (VI) :

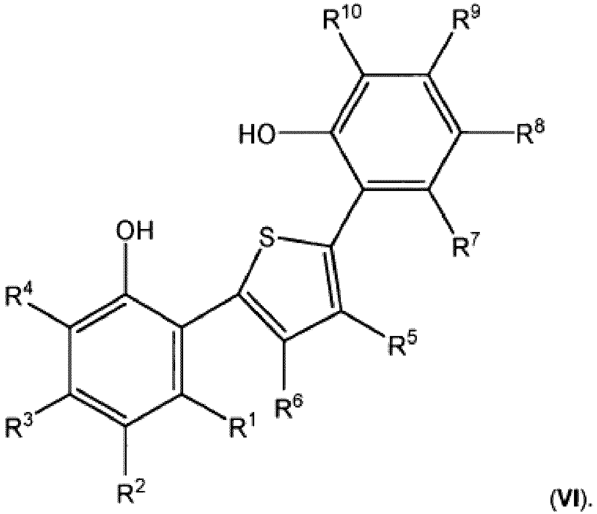

5. Procédé selon l'une quelconque des revendications 1 à 4,
le procédé comprenant l'étape de procédé supplémentaire f) :
f) ajout de méthanol.

6. Procédé selon l'une quelconque des revendications 1 à 5,
l'anode étant une anode BDD (boron doped diamond - diamant dopé au bore).

7. Procédé selon l'une quelconque des revendications 1 à 6,
la cathode étant une cathode BDD.

8. Procédé selon l'une quelconque des revendications 1 à 7,
le procédé comprenant l'étape de procédé supplémentaire g) :
g) ajout de MTBS.
